(19) Europäisches Patentamt
European Patent Office
Office européen des brevets

(11) Publication number : **0 379 309 B1**

(12) **EUROPEAN PATENT SPECIFICATION**

(45) Date of publication of patent specification : **29.03.95 Bulletin 95/13**

(51) Int. Cl.$^6$ : **G03C 7/36**

(21) Application number : **90300328.3**

(22) Date of filing : **11.01.90**

(54) **Benzoylacetanilide photographic yellow dye image-forming couplers and photographic elements containing them.**

(30) Priority : **16.01.89 GB 8900868**

(43) Date of publication of application : **25.07.90 Bulletin 90/30**

(45) Publication of the grant of the patent : **29.03.95 Bulletin 95/13**

(84) Designated Contracting States : **AT BE CH DE DK ES FR GB GR IT LI LU NL SE**

(56) References cited :
**EP-A- 0 317 983**
**EP-A- 0 327 348**
**PATENT ABSTRACTS OF JAPAN vol. 12, no. 62 (P-670)(2909) 25 February 1988, & JP-A-62 204259**

(73) Proprietor : **EASTMAN KODAK COMPANY**
**343 State Street**
**Rochester, New York 14650-2201 (US)**
(84) **BE CH DE DK ES FR GR IT LI LU NL SE AT**
Proprietor : **KODAK LIMITED**
**P.O. Box 66**
**Station Road**
**Hemel Hempstead Herts, HP1 1JU (GB)**
(84) **GB**

(72) Inventor : **Tsoi, Siu Chung**
**89 Cassiobury Drive**
**Watford, Hertfordshire WD1 3AG (GB)**

(74) Representative : **Baron, Paul Alexander Clifford et al**
**Kodak Limited**
**Patent Department**
**Headstone Drive**
**Harrow Middlesex HA1 4TY (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to benzoylacetanilide photographic yellow dye image-forming couplers and to photographic elements containing them.

Couplers of the benzoylacetanilide class are known which contain a heterocyclic coupling.-off group, for example as described in US Patents 4,230,851, 4,327,175 and 4,529,691. Such couplers have good solubility in conventional coupler solvents.

Recently, benzoylacetanilide couplers having aryloxy coupling-off groups have been of interest as providing image dyes of excellent hue and high extinction coefficient. These couplers, however, do tend to be less soluble in coupler solvents than is desirable.

A coupler of this class has been generally described in US patent 4,511,649 (coupler (10)), whose ballast group is attached to the anilide ring of the coupler via an ester group, the moiety having the formula -CO-O-$C_{12}H_{25}$.

Couplers having such a ballast group have less than the desired solubility in coupler solvents.

EP-A-0 317 983 and JP-A-62 204 259 disclose a yellow coupler having a ballast group of the formula:

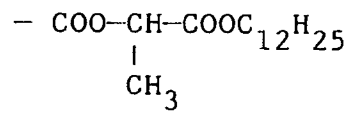

as a coupler providing a photographic material having high sensitivity, sharpness, colour reproducability and preservability.

EP-A-0 327 348 discloses benzoylacetanilide couplers complying with general formulae (I) and (II) shown below. The alkyl chain of the ballast group, which may be interrupted by hereto atoms or groups, must, however, be branched.

Each of EP-A-0 317 983 and EP-A- 0 327 348 was published after present priority date and therefore is only relevant within the terms of Art. 54(3) EPC.

The present invention provides couplers of the benzoylacetanilide type having an aryloxy coupling-off group having improved solubility in coupler solvents.

According to the present invention there are provided non--diffusible couplers of the general formula:

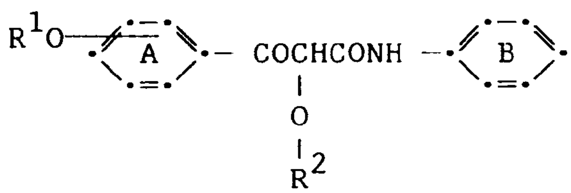

(I)

in which R1

is a cyclic or non-cyclic aliphatic hydrocarbon group which may be substituted or unsubstituted, branched or unbranched,

$R^2$ is an aryl group which may be substituted or unsubstituted,

and wherein at least one of the rings A and B contains a ballasting substituent (which may be $R^1$) comprising a group of the formula:

$$-(CH_2)_p - L^1 - (CH_2)_q - CH_3$$

in which

$L^1$ is -O-CO or -CO-O-, and

p and q are each 1-25.

The present couplers are non-diffusible in photographic elements hence will contain one or more ballasting substituents of sufficient size and configuration to ensure this. As is well understood the necessary bulk may be divided among more than one substituent if desired. The ballast groups may form part of $R^1$ and/or may be directly or indirectly linked to one or both of rings (A) and (B).

The aryl group $R^2$ may be substituted with one or more substituents selected from alkyl, amide, ester, carbamoyl, sulphonamide, sulphamoyl, sulphone, ether, thioether, nitrile, nitro groups and halogen atoms in any position.

Examples of ballast groups which may be employed in the present couplers are: $-(CH_2)_2-O-CO-C_{12}H_{25}-n$, $-(CH_2)_2-O-CO-C_7H_{15}-n$, $-(CH_2)_{10}-CO-O-C_2H_5-n$.

A preferred group of couplers according to the present invention have the general formula:

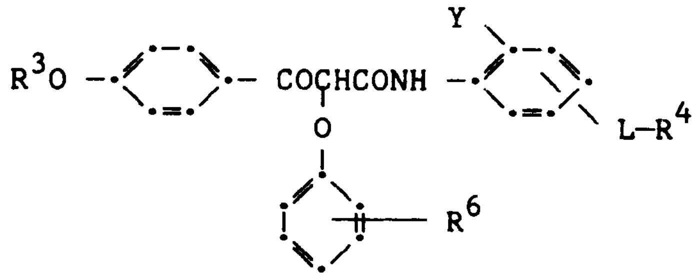

(II)

wherein

R3 and $R^4$ are each a substituted or unsubstituted alkyl group,

Y is chloro or trifluoromethyl,

L is -COO-, -OCO-, $-NR^7CO-$, $-CONR^7$, $-NR^7SO_2$, $-SONR^7$, -O-, $-OSO_2$ or a single bond,

$R^6$ is one or more substituent selected from alkyl, amide, carbamoyl, sulphonamide, sulphamoyl, sulphone, ester, ether, thioether, nitrile, nitro groups and halogen atoms,

$R^7$ is H or alkyl,

and in which the ballast group is constituted by $R^3$ and/or $R^4$ and when R4 is not a ballast group, $-LR^4$ may also be trifluoromethyl, chloro, $-NHPO(OR)_2$ or $-PO(OR)_2$ wherein R is alkyl or aryl.

Examples of the groups which $R^1$ and $R^3$ may represent are alicyclic groups of 5 to 6 carbon atoms, and non-cyclic groups of 1-20 carbon atoms which may be substituted or unsubstituted, saturated or unsaturated, e.g. methyl, ethyl, n-butyl, n-dodecyl, n-hexadecyl, n-undecyl and benzyl.

Examples of coupling-off groups ($R^2O$- in formula (I)) are shown below together with the names by which they will be identified herein;

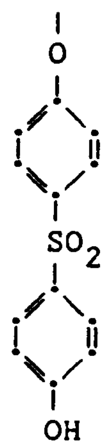

(SDP)

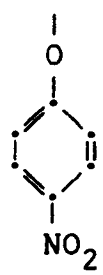

(NP)

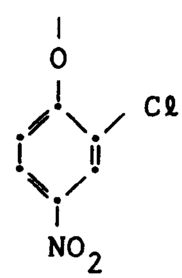

(CNP)

The dye-forming couplers of this invention can be used in the ways and for the purposes that dye-forming couplers have been previously used in the photographic art.

Typically, the couplers are associated with a silver halide emulsion layer coated on a support to form a photographic element. As used herein, the term "associated with" signifies that the coupler is incorporated in the silver halide emulsion layer or in a layer adjacent thereto where, during processing, it is capable of reacting with silver halide development products.

Typically the coupler is dissolved in a coupler solvent and this solution is dispersed in an aqueous gelatin solution. Examples of coupler solvents that may be used are dibutyl phthalate, tricresyl phosphate, diethyl lauramide and 2,4-di-tertiaryamylphenol. In addition an auxilliary coupler solvent may also be used, for example ethyl acetate, cyclohexanone, and 2-(2-butoxyethoxy)ethyl acetate, which are removed from the dispersion before incorporation into the photographic material.

The photographic elements can be single colour elements or multicolour elements. In a multicolour element, the yellow dye-forming couplers of this invention would usually be associated with a blue-sensitive emulsion, although they could be associated with an emulsion sensitised to a different region of the spectrum, or with a panchromatically sensitised, orthochromatically sensitised or unsensitised emulsion. Multicolour elements contain dye image-forming units sensitive to each of the three primary regions of the spectrum. Each unit can be comprised of a single emulsion layer or of multiple emulsion layers sensitive to a given region of the spectrum. The layers of the elements, including the layers of the image-forming units, can be arranged in various orders as known in the art.

A typical multicolour photographic element comprises a support bearing a yellow dye image-forming unit comprised of at least one blue-sensitive silver halide emulsion layer having associated therewith at least one yellow dye-forming coupler being a coupler of this invention, and magenta and cyan dye image-forming units comprising at least one green- or red-sensitive silver halide emulsion layer having associated therewith at least one magenta or cyan dye-forming coupler respectively. The element can contain additional layers, such as filter layers.

In the following discussion of suitable materials for use in the emulsions and elements of this invention, reference will be made to Research Disclosure, December 1978, Item 17643, published by Industrial Opportunities Ltd., The Old Harbourmaster's, 8 North Street, Emsworth, Hants PO10 7DD, U.K. This publication will be identified hereafter as "Research Disclosure".

The silver halide emulsion employed in the elements of this invention can be either negative-working or positive-working. Suitable emulsions and their preparation are described in Research Disclosure Sections I and II and the publications cited therein. Suitable vehicles for the emulsion layers and other layers of elements of this invention are described in Research Disclosure Section IX and the publications cited therein.

In addition to the couplers of this invention, the elements of the invention can include additional couplers as described in Research Disclosure Section VII, paragraphs D, E, F and G and the publications cited therein. The couplers of this invention and any additional couplers can be incorporated in the elements and emulsions as described in Research Disclosure Section VII, paragraph C and the publications cited therein.

The photographic elements of this invention or individual layers thereof, can contain brighteners (see Research Disclosure Section V), antifoggants and stabilisers (see Research Disclosure Section VI), antistain agents and image dye stabiliser (see Research Disclosure Section VII, paragraphs I and J), light absorbing and scattering materials (see Research Disclosure Section VIII), hardeners (see Research Disclosure Section XII, plasticisers and lubricants (see Research Disclosure Section XIII), matting agents (see Research Disclosure Section XVI) and development modifiers (see Research Disclosure Section XXI).

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the elements with a colour developing agent to reduce developable silver halide and oxidise the colour developing agent. Oxidised colour developing agent in turn reacts with the coupler to yield a dye.

Preferred colour developing agents are phenylene diamines. Especially preferred are 4-amino-3-methyl-N,N-diethylaniline hydrochloride, 4-amino-3-methyl-N-ethyl-N-β-(methylanesulphonamido)ethylaniline sulphate hydrate, 4-amino-3-methyl-N-ethyl-N-β-(methanesulphonamido)ethyl-N,N-diethylaniline hydrochloride and 4-amino-N-ethyl-N-(2-methoxyethyl)-m-toluidine di-p-toluene sulphonate.

With negative-working silver halide emulsions this processing step leads to a negative image. To obtain a positive (or reversal) image, this step can be preceded by development with a non-chromagenic developing agent to develop exposed silver halide, but not form dye, and then uniform fogging of the elements to render unexposed silver halide developable. Alternatively, a direct positive emulsion can be employed to obtain a positive image.

Development is followed by the conventional steps of bleaching, fixing, or bleach-fixing, to remove silver and silver halide, washing and drying.

Specific examples of couplers according to the present invention are listed in Table I below.

TABLE I

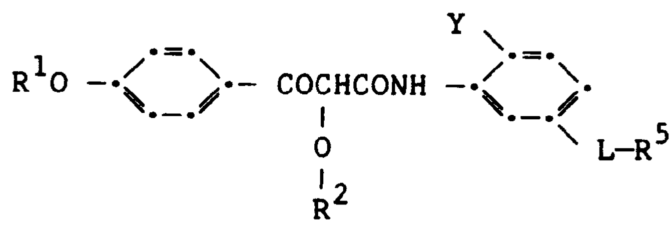

| Coupler | $R^1$ | $L-R^5$ | $OR^2$ |
|---|---|---|---|
| 1 | $-CH_3$ | $-COO(CH_2)_2O-COC_{12}H_{25}-n$ | SDP |
| 2 | $-(CH_2)_{10}COOC_2H_5$ | $-Cl$ | SDP |
| 3 | $-(CH_2)_{10}COOC_2H_5$ | $-CF_3$ | SDP |
| 4 | n-BuO | $-CO_2CH_2CH_2OCO(CH_2)_6CH_3$ | SDP |

The present couplers may be prepared by methods in themselves known. A specific preparation is given below.

The following Examples are included for a better understanding of the invention.

Example 1

Solubility Test for Couplers

Each test coupler (0.24g) together with di-n-butylphthalate (0.12g) and ethyl acetate (0.72g) was accurately weighed into a standard test tube with a plug of cotton wool at the top. The samples were placed in a preheated water bath at 75-80°C, with occasional agitation, for 15 minutes and then allowed to stand at room temperature. The samples were examined for crystallisation (or sign of opacity) at the following time intervals; 5, 10, 15, 20, 25, 30, 45, 60, 90 and 120 minutes after being removed from the water bath.

The results are given in Table II together with those of comparative couplers of the prior art (whose full structures are given in Table III). It can be seen that the couplers of the present invention exhibit superior solubility over their straight chain counterparts.

The test is considered to be a good indication of how a coupler will behave in a photographic coating.

## TABLE II

| Coupler | Ballast Group | Time remained as a clear solution (min) |
|---|---|---|
| 1 | $-COO(CH_2)_2O-COC_{12}H_{25}$-n | >120 |
| C1 | $-COOC_{14}H_{29}$-n | 20-25 |
| C2 | $-COOC_{16}H_{33}$-n | 45-60 |
| 2 | $-(CH_2)_{10}-COOC_2H_5$ | 90-120 |
| C3 | $-C_{12}H_{25}$-n | 20-25 |
| 3 | $-(CH_2)_{10}-COOC_2H_5$ | 60-90 |
| C4 | $-C_{12}H_{25}$-n | 0 |
| 4 | $-COO-(CH_2)_2-OCO(CH_2)_6CH_3$ | >120 (glass) |
| C5 | $-COOC_{12}H_{25}$-n | <5 |

## TABLE III

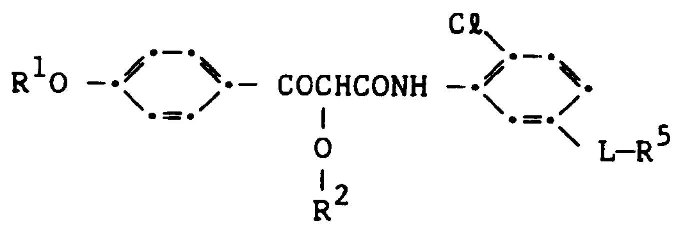

(I)

| Coupler | $R^1$ | $-L-R^5$ | $-OR^2$ |
|---|---|---|---|
| C1 | $-CH_3$ | $-COOC_{14}H_{29}$-n | SDP |
| C2 | $-CH_3$ | $-COOC_{16}H_{33}$-n | SDP |
| C3 | $-C_{12}H_{25}$-n | $-C\ell$ | SDP |
| C4 | $-C_{12}H_{25}$-n | $-CF_3$ | SDP |
| C5 | -Bu-n | $-CO_2C_{12}H_{25}$-n | SDP |

Example 2

Preparation of Coupler 3 of Table 1

(a) Ethel 11-bromoundecanoate

A solution of 11-bromoundecanoic acid (53g, 0.2 mol) and concentrated sulphuric acid (1ml) in ethanol (250 ml) was heated to reflux for 24 hr. After cooling to room temperature, the mixture was concentrated to half its original volume by evaporation under reduced pressure. The residue was poured into water (400 ml) and extracted with diethyl ether (3 x 300 ml). The combined extracts were then washed with saturated sodium bicarbonate solution (3 x 200 ml) and water (3 x 200 ml). After drying over $MgSO_4$ the solvent was evaporated under reduced pressure to give a yellow oil (55.5g, 95%)

(b) Ethyl 11-(4-acetoxyphenoxy)undecanoate

A mixture of the bromoester from (a) (55.3g, 0.19mol), 4-hydroxyacetophenone (25.7g, 0.19 mol) and potassium carbonate (26.1g, 0.19 mol) in acetone (250ml) was heated to reflux with stirring for 2 days. After cooling to room temperature, water (500 ml) was added and the whole mixture extracted with ethyl acetate (3 x 300 ml). The combined extracts were then washed with 3N sodium hydroxide solution (300ml), water (2 x 300 ml), 3N HCl solution (200ml) and water (2 x 300 ml). After drying over $MgSO_4$ the solvent was removed by evaporation under reduced pressure and the residue recrystallised from methanol to give a white solid (42.9g, 65%).

Found C, 72.33; H, 9.41;

$C_{21}H_{32}O_4$ requires; C, 72.36; H, 9.26%

(c) Ethyl 11-(4-ethoxycarbonylacetoxyphenoxy)undecanoate

The product from (b) (42g, 0.12 mol) was added portionwise to a suspension of potassium t-butoxide (21.3g, 0.19 mol) in diethyl carbonate (56.6g, 0.48 mol) at 75°C over 10 minutes. Heating was continued at 95°C for 1 h. After cooling to 50°C, the suspension was poured into cold water (1 litre) and extracted with ethyl acetate (3 x 400 ml). The organic solution was dried and evaporated under reduced pressure to give the product (47g, 93%) as a yellow waxy solid which was used in the next reaction without further purification.

(d) Ethyl 11-{4-[2-(2-chloro-5-trifluoromethylphenylcarbamoyl)acetoxy]phenoxy}undecanoate

A solution of the product from (c) (47g, 0.112 mol) and 3-amino-4-chlorobenzotrifluoride (21.9g, 0.112 mol) in xylene (450 ml) were heated to reflux for 5 h. Over the final hour, the volume of the mixture was reduced to 200 ml by distillation. After cooling to about 80°C, the solution was poured into petrol (b.p. 60-80°C (1 litre) with rapid stirring. A pale yellow solid was collected by filtration and then recrystallised from petrol (b.p. 60-80°C) to give an off white solid (40.5g, 63%).

(e) Ethyl 11-{4-[2-chloro-2-(2-chloro-5-trifluoromethylphenylcarbamoyl)acetoxy]phenoxy}undecanoate

Sulphuryl chloride (9.5g, 70.2 m mol) in dichloromethane (15 ml) was slowly added to a solution of the 4-equivalent coupler from (d) (40g, 70.2 m mol) in dichloromethane (250 ml). After stirring at room temperature for 20h, the volatiles were evaporated under reduced pressure. The crude product was purified by silica gel column chromatography (elutant 10% ethyl acetate in petrol (b.p. 60-80°C)) followed by recrystallisation from methanol to give a white solid (25.2g, 60%).

Found C, 57.67; H, 5.56; Cl, 11.60; F, 9.47; N,2.33

$C_{29}H_{34}Cl_2F_3NO_5$ requires; C, 57.62; H, 5.67; Cl, 11.73; F, 9.43; N, 2.32%

(f) Ethyl 11-{4-[2-(2-chloro-5-trifluoromethylphenylcarbamoyl)-2-(4-((4-hydroxyphenylsulphonyl))phenoxy) acetoxy]phenoxy}undecanoate

Triethylamine (12. 12g, 120 mmol) was added to a solution of the chloro-coupler (24.5g, 40.5 mmol) from (e) and 4,4'-sulfonyldiphenol (40.5g, 162 mmol) in dry DMF (130 ml) which has been degassed with nitrogen. After stirring at 45-50°C under nitrogen for 1 h, the suspension was cooled and poured slowly into cold water (300 ml) and conc. hydrochloric acid (10 ml), with rapid stirring. The whole mixture was extracted with ethyl acetate (500 ml) and the organic solution washed with 3N sodium carbonate (2 x 400 ml), 3N hydrochloric acid (300 ml), dried over $MgSO_4$ and evaporated under reduced pressure. The crude material was purified by silica gel column chromatography (gradient elution from 20% ethyl acetate in petrol (b.p. 60-80°C) to 60% ethyl acetate in petrol (b.p. 60-80°C) followed by recrystallisation from methanol to give a white solid (13.5g, 41%).

Found C, 60.29; H, 5.25; Cl, 4.16; F, 6.98; N, 1.69; S, 3.91

$C_{41,}H_{43}ClF_3NO_9S$ requires; C, 60.18; H, 5.30; Cl, 4.33; F, 6.97; N, 1.71; S, 3.92%.

## Claims

1. A non-diffusible coupler of the general formula:

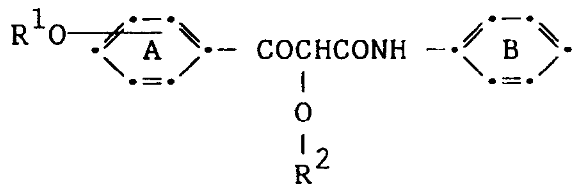 (I)

in which
$R^1$ is a cyclic or non-cyclic aliphatic hydrocarbon group which may be substituted or unsubstituted, branched or unbranched,
$R^2$ is an aryl group which may be substituted or unsubstituted,
and wherein at least one of the rings A and B contains a ballasting substituent (which may be $R^1$) comprising a group of the formula:

$$-(CH_2)_p - L^1 - (CH_2)_q - CH_3 \qquad (Ia)$$

in which
$L^1$ is -O-CO or -CO-O-, and
p and q are each 1-25.

2. A coupler as claimed in Claim 1 in which the aryl group $R^2$ is substituted with one or more substituents selected from alkyl, amide, ester, carbamoyl, sulphonamide, sulphamoyl, sulphone, ether, thioether nitrile, nitro groups and halogen atoms in any position.

3. A coupler as claimed in Claim 1 or 2 comprising one of the ballast groups:
$-(CH_2)_2-O-CO-C_{12}H_{25}-n$, $-(CH_2)_2-O-CO-C_7H_{15}-n$, $-(CH_2)_{10}-CO-O-C_2H_5-n$.

4. A coupler according to Claim 1 having the general formula:

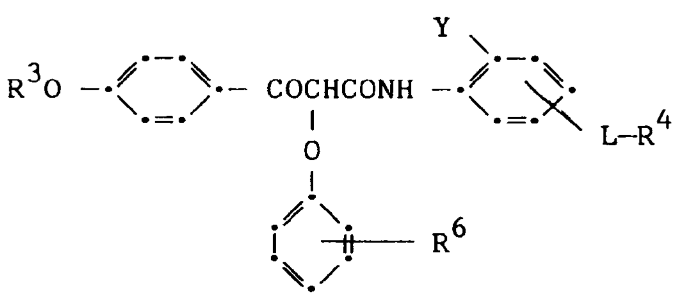 (II)

wherein
R3 and $R^4$ are each a substituted or unsubstituted alkyl group,
Y is chloro or trifluoromethyl,
L is -COO-, -OCO-, $-NR^7CO-$, $-CONR^7$, $-NR^7SO_2$, $-SONR^7$, -O-, $OSO_2$ or a single bond,
$R^6$ is one or more substituent selected from alkyl, amide, carbamoyl, sulphonamide, sulphamoyl, sulphone, ester, ether, thioether, nitrile, nitro groups and halogen atoms,
$R^7$ is H or alkyl,
and in which the ballast group of formula (Ia) is constituted by $R^3$ and/or $R^4$ and when R4 is not a ballast group, $-LR^4$ may also be trifluoromethyl, chloro, $-NHPO(OR)_2$ or $-PO(OR)_2$ wherein R is alkyl or aryl.

5. A coupler as claimed in Claim 4 in which $R^3$ is an alicyclic group of 5-6 carbon atoms or a non-cyclic group of 1-20 carbon atoms which may be substituted or unsubstituted, saturated or unsaturated.

6. A photosensitive photographic material comprising a support bearing a silver halide emulsion layer having associated therewith a dye-forming coupler according to any of Claims 1-5.

7. A photosensitive material as claimed in Claim 6 which is a multicolour photographic element comprising

a support bearing a yellow dye image-forming unit comprised of at least one blue-sensitive silver halide emulsion layer having associated therewith at least one yellow dye-forming coupler according to any of Claims 1-5, and magenta and cyan dye image-forming units comprising at least one green- or red-sensitive silver halide emulsion layer having associated therewith at least one magenta or cyan dye-forming coupler respectively.

## Patentansprüche

1. Nicht-diffundierender Kuppler der allgemeinen Formel:

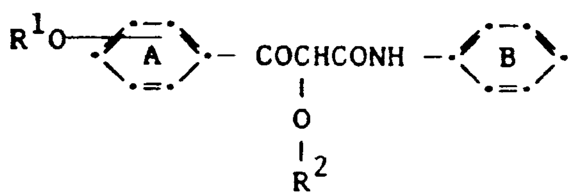

(I)

worin bedeuten: $R^1$ eine cyclische oder nicht-cyclische aliphatische Kohlenwasserstoffgruppe, die substituiert oder unsubstituiert, verzweigt oder unverzweigt sein kann,
$R^2$ eine Arylgruppe, die substituiert oder unsubstituiert sein kann,
und wobei mindestens einer der Ringe A und B einen Ballast-Substituenten aufweist (der $R^1$ sein kann) mit einer Gruppe der Formel:

$$-(CH_2)_p - L^1- (CH_2)_q-CH_3 \qquad (Ia)$$

worin $L^1$ steht für -O-CO oder -CO-O-, und worin bedeuten
p und q jeweils 1 - 25.

2. Kuppler nach Anspruch 1, in dem die Arylgruppe $R^2$ substituiert ist durch einen oder mehrere Substituenten, die ausgewählt sind aus Alkyl-, Amid-, Ester-, Carbamoyl-, Sulfonamid-, Sulfamoyl-, Sulfon-, Ether-, Thioether-, Nitril- und Nitrogruppen und Halogenatomen in jeder beliebigen Position.

3. Kuppler nach Ansprüchen 1 oder 2, der eine der Ballastgruppen:
$-(CH_2)_2-O-CO-C_{12}H_{25}$-n, $-(CH_2)_2-O-CO-C_7H_{15}$-n, $-(CH_2)_{10}-CO-O-C_2H_5$-n enthält.

4. Kuppler nach Anspruch 1, mit der allgemeinen Formel:

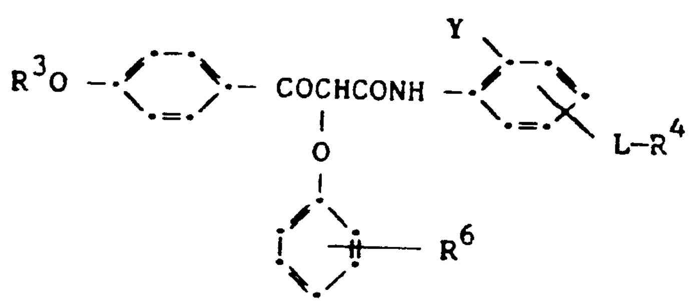

(II)

worin bedeuten: $R^3$ und $R^4$ jeweils eine substituierte oder unsubstituierte Alkylgruppe,
Y gleich Chloro oder Trifluoromethyl,
L gleich -COO-, -OCO-, $-NR^7CO-$, $-CONR^7$, $-NR^7SO_2$, $-SONR^7$, -O-, $-OSO_2$ oder eine einfache Bindung,
$R^6$ einen oder mehrere Substituenten, ausgewählt aus Alkyl-, Amid-, Carbamoyl-, Sulfonamid-, Sulfamoyl-, Sulfon-, Ester-, Ether-, Thioether-, Nitril- und Nitrogruppen und Halogenatomen,
$R^7$ gleich H oder Alkyl, und
worin die Ballastgruppe der Formel (Ia) gebildet wird durch $R^3$ und/oder $R^4$, und wobei gilt, daß, wenn $R^4$ keine Ballastgruppe ist, $-LR^4$ auch stehen kann für Trifluoromethyl, Chloro, $-NHPO(OR)_2$ oder $-PO(OR)_2$, worin R für Alkyl oder Aryl steht.

5. Kuppler nach Anspruch 4, worin $R^3$ eine alicyclische Gruppe mit 5 - 6 Kohlenstoffatomen ist oder eine

nicht-cyclische Gruppe mit 1 - 20 Kohlenstoffatomen, die substituiert oder unsubstituiert, gesättigt oder ungesättigt sein kann.

6. Photosensitives photographisches Material mit einem Träger, auf dem sich eine Silberhalogenidemulsionsschicht befindet, der ein einen Farbstoff bildender Kuppler nach einem der Ansprüche 1 - 5 zugeordnet ist.

7. Photosensitives Material nach Anspruch 6, bestehend aus einem mehrfarbigen photographischen Element mit einem Träger, auf dem sich eine ein gelbes Farbstoffbild erzeugende Einheit befindet mit mindestens einer blau-empfindlichen Silberhalogenidemulsionsschicht, der mindestens ein einen gelben Farbstoff bildender Kuppler nach einem der Ansprüche 1 - 5 zugeordnet ist, und mit purpurrote und blaugrüne Farbstoffbilder liefernden Einheiten mit mindestens einer grün- oder rot-empfindlichen Silberhalogenidemulsionsschicht, der mindestens ein einen purpurroten Farbstoff bzw. einen blaugrünen Farbstoff liefernder Kuppler zugeordnet ist.

## Revendications

1. Coupleur non-diffusible de formule générale :

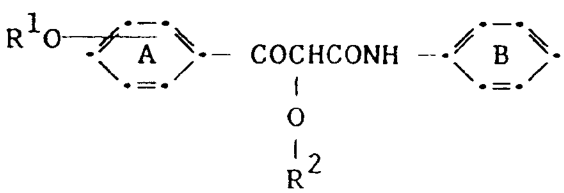

(I)

dans lequel

$R^1$ est un groupe hydrocarboné aliphatique cyclique ou non-cyclique qui peut être substitué ou non, ramifié ou non,

$R^2$ est un groupe aryle substitué ou non, et dans lequel au moins un des noyaux A et B contient un substituant ballastant (qui peut être $R^1$) comprenant un groupe de formule :

$$-(CH_2)_p - L^1 - (CH_2)_q - CH_3 \qquad (Ia)$$

dans laquelle $L^1$ est -O-CO ou -CO-O-, et p et q sont chacun de 1 à 25.

2. Coupleur selon la revendication 1 dans lequel le groupe aryle $R^2$ est substitué dans n'importe quelle position par un ou plusieurs substituants choisis parmi les groupes alkyle, amide, ester, carbamyle, sulfonamide, sulfamyle, sulfone, éther, thioéther, nitrile, nitro et les atomes d'halogène.

3. Coupleur selon la revendication 1 ou 2 comprenant l'un des groupes ballast :
$-(CH_2)_2-O-CO-C_{12}H_{25}-n$, $-(CH_2)_2-OCO-C_7H_{15}-n$, $-(CH_2)_{10}-CO-O-C_2H_5-n$.

4. Coupleur selon la revendication 1 de formule générale :

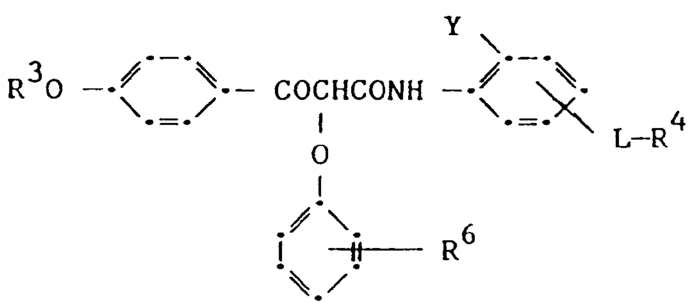

(II)

où

$R^3$ et $R^4$ sont chacun un groupe alkyle substitué ou non,

Y est chloro ou trifluorométhyle,

L est -COO-, -OCO-, $-NR^7CO-$, $-CONR^7$, $-NR^7SO_2$, $-SONR^7$, -O-, $-OSO_2$ ou une liaison simple,

$R^6$ est un ou plusieurs substituants choisis parmi les groupes alkyle, amide, ester, carbamyle, sulfonamide, sulfamyle, sulfone, éther, thioéther, nitrile, nitro et les atomes d'halogène

$R^7$ est H ou alkyle,

et dans lequel le groupe ballast de formule (Ia) est constitué par $R^3$ et/ou $R^4$ et quand $R^4$ n'est pas un groupe ballast, $-LR^4$ peut aussi être trifluorométhyle, chloro, $-NHPO(OR)_2$ ou $-PO(OR)_2$ où R est alkyle ou aryle.

**5.** Coupleur selon la revendication 4 dans lequel $R^3$ est un groupe alicyclique de 5 à 6 atomes de carbone ou un groupe non-cyclique de 1 à 20 atomes de carbone qui peut être substitué ou non, saturé ou non.

**6.** Matériau photographique photosensible comprenant un support recouvert d'une couche d'émulsion aux halogénures d'argent à laquelle est associé un coupleur formateur de colorant selon l'une quelconque des revendications 1 à 5.

**7.** Matériau photosensible selon la revendication 6 qui est un produit photographique multicolore comprenant un support recouvert d'une unité formatrice de colorant jaune comprenant au moins une couche d'émulsion aux halogénures d'argent sensible au bleu à laquelle est associé au moins un coupleur formateur de colorant jaune selon l'une quelconque des revendications 1 à 5 et des unités formatrices de colorant cyan et magenta comprenant au moins une couche d'émulsion sensible au vert ou au rouge à laquelle est associé respectivement au moins un coupleur formateur de colorant magenta ou cyan.